(19) Europäisches Patentamt
European Patent Office
Office européen des brevets

(11) **EP 1 626 275 B1**

## (12) EUROPÄISCHE PATENTSCHRIFT

(45) Veröffentlichungstag und Bekanntmachung des Hinweises auf die Patenterteilung:
**12.09.2007 Patentblatt 2007/37**

(51) Int Cl.:
***G01N 33/02*** *(2006.01)*

(21) Anmeldenummer: **05014480.7**

(22) Anmeldetag: **04.07.2005**

(54) **Verfahren zur Bewertung der Produkthaltbarkeit in einem Packmittel**

Method for determining the shelf-life of a packed product

Procédé pour déterminer la durée de conservation d'un produit emballé

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **12.08.2004 DE 102004039210**

(43) Veröffentlichungstag der Anmeldung:
**15.02.2006 Patentblatt 2006/07**

(73) Patentinhaber: **RUDOLF WILD GmbH & CO. KG**
**69052 Heidelberg (DE)**

(72) Erfinder:
• **Wild, Hans Peter, Dr.**
**69214 Eppelheim (DE)**
• **Küssner, Klaus**
**69251 Gaiberg (DE)**
• **Spinner, Frank**
**76228 Karlsruhe (DE)**

(74) Vertreter: **Grünecker, Kinkeldey,**
**Stockmair & Schwanhäusser**
**Anwaltssozietät**
**Maximilianstrasse 58**
**80538 München (DE)**

(56) Entgegenhaltungen:
DE-A1- 10 240 295          US-A1- 2004 007 072
US-A1- 2004 040 372          US-B1- 6 640 615

• **PETERSEN M A ET AL: "Comparison of normal and accelerated storage of commercial orange juice - changes in flavour and content of volatile compounds." FOOD QUALITY AND PREFERENCE 1998 DEP. OF DAIRY & FOOD SCI./ CENT. FOR ADVANCED FOOD STUDIES, ROYAL VET. & AGRIC. UNIV., DK-1958 FREDERIKSBERG C, DENMARK. FAX +45 35 28 32 65. E-MAIL MAP (A)KVL.D, Bd. 9, Nr. 1/2, 25. Februar 1998 (1998-02-25), Seite 43, XP002378268**

EP 1 626 275 B1

**Beschreibung**

Die Erfindung betrifft ein Verfahren zur Bewertung der Produkthaltbarkeit in einem Packmittel, sowie die Verwendung einer Vorrichtung zum Beschleunigen der Permeation eines Testgases in ein Packmittel für dieses Verfahren.

Aus der US 2004/049372 A1 ist bereits eine Testvorrichtung bekannt mit einem Überdruckbehälter, einem Gaseingang zum Zuführen von Testgas, einem Gasausgang zum Ableiten von Testgas und einer Einrichtung zum Einstellen und Halten des Überdrucks.

Insbesondere im Lebensmittelbereich müssen die unterschiedlichen Verpackungen einen optimalen Produktschutz gewähren. Die Packmittel müssen dabei sowohl im Hinblick auf die Materialauswahl als auch das Verpackungsdesign hohen packgutspezifischen Anforderungen genügen. Innerhalb der deklarierten Mindesthaltbarkeit muss eine einwandfreie Qualität des Produkts gewährleistet sein. Neben der mikrobiologischen Haltbarkeit, die durch geeignete Verfahren, wie Erwärmen, aseptisches Abfüllen und Verpackung, sowie Konservieren gewährleistet werden kann, kommt es auch wesentlich auf die chemischen oder physikalischen Veränderungen des Füllgutes, die zu Aromaveränderungen führen können, an. Insbesondere die Permeation von Stoffen, wie insbesondere Sauerstoff, durch das Packmittel und die subsequente Veränderung der Zusammensetzung des Packgutes durch diese Stoffe kann die Qualität des Produktes in entscheidender Weise beeinflussen.

Zur Ermittlung der Mindesthaltbarkeit stehen neben Materialprüfungstests und theoretischen Berechnungsmodellen auch Standardqualifikationstests, wie etwa Produktlagertest in Echtzeit zur Verfügung. Dabei werden die zu untersuchenden Packmittel abgefüllt und über den Zeitraum der dafür vorgesehenen Mindesthaltbarkeit unter kontrollierten Bedingungen gelagert. Dies bedeutet allerdings, dass eine hinreichend gesicherte Aussage über die Tauglichkeit eines Verpackungsdesigns für ein bestehendes Produkt oder im Umkehrschluss einer Produktneuentwicklung für eine bestehende Verpackungsart erst nach Ablauf der vorgesehenen Mindesthaltbarkeit getroffen werden kann. In dieser sehr langen Testzeit von mehreren Monaten zeigt sich der wesentliche Nachteil dieser Testmethode. Bedingt durch die immer größer werdende Produktvielfalt, immer kürzer werdenden Produktzyklen bei gleichzeitig hoher Erwartung des Verbrauchers bezüglich Produktqualität und -sicherheit besteht im Wettbewerb der Anbieter von Produkten die Notwendigkeit zur Reduzierung der benötigten Entwicklungszeit.

Hiervon ausgehend liegt der vorliegenden Erfindung die Aufgabe zugrunde, ein Verfahren zur Bewertung der Produkthaltbarkeit in einem Packmittel, sowie zum Beschleunigen der Permeation eines Testgases in ein Packmittel bereitzustellen, das die Testzeit wesentlich reduziert und realistische Ergebnisse der zeitabhängigen Permeation in das Füllgut auf einfache Art und Weise liefert.

Erfindungsgemäß wird diese Aufgabe durch die Merkmale der Ansprüche 1 und 12 gelöst.

Gemäß der vorliegenden Erfindung lässt sich der Vorgang der Permeation von Testgas in das Packmittel durch die Erhöhung des Drucks $p_1$ zeitlich beschleunigen. Der Grad der Permeation ist dabei linear abhängig von der realen Permeabilität des Packmittels bei Umgebungsdruck (Normaldruck) in Echtzeit. Durch das anschließende Lagern des Packmittels über einen bestimmten Zeitraum unter Wärme und/oder Lichteinfluss kann die Interaktion des durch die Forcierung in größerem Maße vorliegenden Gasgehalts mit anderen Inhaltsstoffen der Produkte stattfinden. So entsteht ein realistischer Eindruck einer mehrmonatigen Lagerung. Aromaveränderungen, die durch die kombinierte Einwirkung von Licht und Sauerstoff entstehen, können durch eine zusätzliche Lichtexposition simuliert werden. Das erfindungsgemäße Verfahren ermöglicht ein rasches Bewertungsverfahren, das in einer oder wenigen Wochen durchgeführt werden kann und somit im Vergleich zu Echtzeittests, die mehrere Monate benötigen, zu einer deutlichen Zeitersparnis führt. Somit kann auf einfache Art und Weise und in kurzer Zeit die Kompatibilität eines bestehenden Produktes zu einem bestimmten Packmittel im Hinblick auf eine optimale Produktqualität bestimmt werden.

Gemäß einem bevorzugten Ausführungsbeispiel der vorliegenden Erfindung umfasst das Testgas $O_2$ oder besteht aus reinem $O_2$. Somit kann die durch die Gaspermeabilität des Packmittels bedingte zeitabhängige Aufnahme von Sauerstoff durch das Füllgut im Zuge der Lagerung simuliert werden.

Vorzugsweise liegt die Temperatur $T_1$ im Schritt b) in einem Bereich von 5 bis 40° C, vorzugsweise bei Zimmertemperatur in einem Bereich von 15 bis 25°C. Der Überdruck $p_1$ liegt vorzugsweise in einem Bereich von 0,1 bis 2,5 MPa.

Da die Permeation auch von dem relativen Feuchtigkeitsgehalt in der Überdruckkammer abhängt, ist es vorteilhaft, auch den relativen Feuchtigkeitsgehalt in der Überdruckkammer einzustellen, vorzugsweise in einem Bereich von 40 bis 50 %. Somit liegen bei den unterschiedlichen Messungen vergleichbare Versuchsparameter vor.

In Schritt c) liegt die Temperatur $T_2$, bei der das Packmittel über einen bestimmten Zeitraum unter Wärme gelagert wird, in einem Bereich von 25 bis 60°C. Somit werden Interaktionen des durch die Forcierung in größerem Maße vorliegenden Testgasgehalts mit anderen Inhaltsstoffen der Produkte beschleunigt und verstärkt.

Gemäß der vorliegenden Erfindung kann zusätzlich ein zu dem Zeitraum $t_1$ äquivalenter Echtlagerzeitraum $t_{echt}$ ermittelt werden, in dem bei Normaldruck die gleiche definierte Menge Q an Testgas in das Packmittel permeiert. Somit ist es möglich in kurzer Zeit, d. h. in wenigen Tagen, eine Aussage über den Produkthaltbarkeitszeitraum zu machen. Alternativ dazu kann auch für Schritt b) die Zeit $t_1$ und der Überdruck $P_1$ sowie die Temperatur $T_1$ so festgelegt werden, dass eine definierte Menge Q an Testgas in das Packmittel permeiert, die der Menge $Q_{echt}$ entspricht, die bei Umgebungsdruck (Normaldruck) in Echtzeit in das Packmittel permeiert. Bei dieser Ausführungsart kann beispielsweise dann in kurzer Zeit herausgefunden werden, ob ein Packmittel für ein bestehendes Produkt geeignet ist.

Dieses Verfahren ist insbesondere geeignet für Kunststoff-Flaschen.

Im Schritt c) liegt der Zeitraum $t_2$ vorzugsweise in einem Bereich von 48 bis 720 Stunden.

Gemäß einem bevorzugten Ausführungsbeispiel wird nach Schritt c) bei carbonisiertem Füllgut der $CO_2$-Gehalt des Füllguts derart eingestellt, dass er dem $CO_2$-Gehalt entspricht, den das Füllgut nach Lagerung in Echtzeit $t_{echt}$ hätte, um für die sensorische Bewertung den $CO_2$-Verlust realitätsnah nachzustellen.

Die Vorrichtung, zum Beschleunigen der Permeation eines Testgages in ein Packmittel die für das erfindungsgemäße Verfahren verwendet wird, ist einfach aufgebaut und kann kostengünstig realisiert werden. Vorzugsweise weist die Vorrichtung eine Heizeinrichtung auf, um das mindestens eine Packmittel auf einer bestimmten Temperatur $T_1$ zu halten, um reproduzierbare Testbedingungen zu gewährleisten. Darüber hinaus kann die Vorrichtung eine Einrichtung zum Einstellen des relativen Feuchtigkeitsgehalts in dem Überdruckbehälter aufweisen, um reproduzierbare Permeationsbedingungen zu erhalten. Der Druckbehälter ist in einem Überdruckbereich von 0 bis 2 MPa, vorzugsweise bis 1 MPa betreibbar.

Gemäß einem bevorzugten Ausführungsbeispiel der vorliegenden Erfindung ist der Gasausgang mit einem Gasmesssystem verbunden, zum Analysieren der atmosphärischen Zusammensetzung des Testgases im Innern des Überdruckbehälters.

Die vorliegende Erfindung wird nachfolgend unter Bezugnahme der begleitenden Zeichnungen näher erläutert.

Figur 1     zeigt eine schematische Darstellung einer Vorrichtung, die für das erfindungsgemäße Verfahren verwendet wird;

Figur 2     zeigt schematisch ein Flussdiagramm einer Ausführungsform des erfindungsgemäßen Verfahrens.

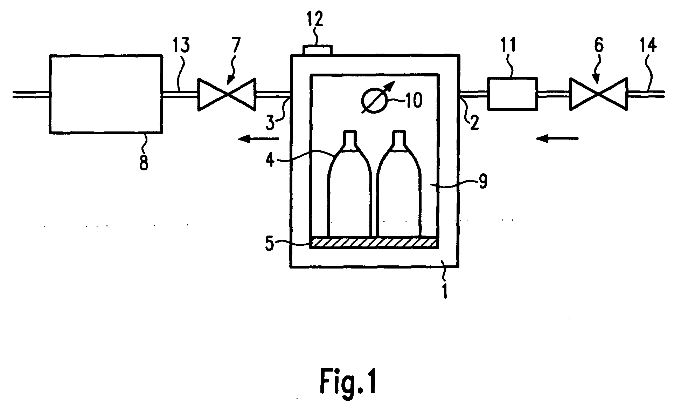

Wie aus Figur 1 ersichtlich ist, umfasst die Vorrichtung einen Überdruckbehälter 1 zur Aufnahme von mindestens einem gefüllten und verschlossenen Packmittel, hier beispielsweise von zwei nebeneinander angeordneten PET-Flaschen 4. Der Überdruckbehälter 1 weist eine verschließbare Einbringöffnung 9 auf. Der Überdruckbehälter 1 ist bis zu einem Druck von 2 MPa betreibbar und weist darüber hinaus eine Überdrucksicherung 12 auf, die bei einem Druck, der oberhalb des Betriebsdrucks liegt, über ein Ventil (nicht dargestellt) den Überdruck abbaut. Darüber hinaus weist die Vorrichtung einen Gaseingang 2 zum Zuführen eines Testgases auf, sowie einen Gasausgang 3 zum Ableiten des Testgases. An den Gaseingang 2 schließt sich eine Zuleitung 14 an und an den Gasausgang 3 schließt sich eine Ableitung 13 an. Die Zuleitung 14 umfasst ein Eingangsventil 6 und die Ableitung 13 umfasst ein Auslassventil 7. Die Vorrichtung umfasst weiter eine Einrichtung 10, 6, 2, 7 zum Einstellen und Halten eines Überdrucks $p_1$ in der Überdruckkammer 1. Der Gasdruckmesser 10 kann dabei, wie in Fig. 1 dargestellt, in der Überdruckkammer angeordnet sein, es ist jedoch auch möglich, den Gasdruckmesser in der Zu- oder Ableitung 13, 14 im Bereich vom Gaseingang oder -ausgang 2, 3vorzusehen. Somit kann beispielsweise über die

Zuleitung 14 bei geöffnetem Einlassventil 6 der Überdruckkammer 1 solange Testgas zugeführt werden bis der Gasdruckmesser 10 einen bestimmten Überdruck $p_1$ anzeigt. Die Vorrichtung kann dazu auch eine nicht gezeigte elektronische Steuerung aufweisen, die den Gasdruck in der Überdruckkammer 1 regelt.

Die Vorrichtung weist vorzugsweise eine Heizeinrichtung 5 auf, die es erlaubt, die Packmittel 4 auf einer vorbestimmten Temperatur $T_1$ zu halten, um reproduzierbare Testbedingungen zu bewerkstelligen.

Weiter umfasst die Vorrichtung eine Einrichtung 11 zum Einstellen der relativen Luftfeuchtigkeit. Da die relative Luftfeuchtigkeit ein Kriterium für die Permeation von Testgas in das Packmittel 4 ist, ist es vorteilhaft, wenn in der Überdruckkammer eine bestimmte relative Luftfeuchtigkeit eingestellt werden kann. Als Einrichtung zum Einstellen der relativen Luftfeuchtigkeit dient beispielsweise ein mit einem Kunststoffvlies gefülltes überdrucksicheres Leitungszwischenstück, das über eine separates Anschlussstück mit z.B. destilliertem Wasser getränkt werden kann. Die aus z.B. mit Hilfe eines Mollier h-X-Diagrammes und dem Wert des zu erzielenden Druckes im Behälter errechnete Menge an Wasser, die zur Einstellung einer definierten relativen Luftfeuchte in der Behälteratmosphäre benötigt wird, kann so auf das Kunststoffvlies aufgebracht werden. Während der Befüllung des Behälters mit Gas durchströmt dieses das Vlies, verdampft das Wasser und führt den Wasserdampf in den Behälter mit.

Bei diesem Ausführungsbeispiel ist der Gasausgang 3 bzw. die Ableitung 13 mit einem Gasmesssystem 8 verbunden, das die atmosphärische Zusammensetzung im Inneren der Überdruckkammer 1 analysiert. Das Gasmesssystem basiert beispielsweise auf elektrochemischer Sensorentechnik und kann mehrere Gase gleichzeitig (z. B. qualitativ und quantitativ) messen (z.B. Dräger Mehrgas-Scanner).

Die Zuleitung 14 ist mit einem nicht gezeigten Gaspaneel verbunden, um ein Testgas oder eine bestimmte Testgasmischung zuzuführen.

Mit der erfindungsgemäßen Vorrichtung kann die Permeation eines Testgases in das Packmittel 4, hier die PET-Flaschen 4, beschleunigt werden. Bei der Permeation in das Packmittel 4 lösen sich Gase und Dämpfe an der Oberfläche des permeablen Packmittels, diffundieren aufgrund des Konzentrationsgefälles durch das Packmittel und gehen in das Füllgut über. Bei der Permeation löst sich der Permeat zunächst im Kunststoff bis sich eine Gleichgewichtskonzentration einstellt (lag-Phase). Anschließend erfolgt die eigentliche Permeation des Testgases linear entsprechend folgender Gleichung (1)

$$Q/t = P/X \; A \; \Delta p.$$

Q ist dabei die Gesamtmenge an Permeat, die in der Zeit t durch eine Kunststoffschicht der Oberfläche A und der Dicke X durchgeht, $\Delta p$ die Partialdruckdifferenz des Permeats zwischen Außen- und Innenseite des Kunststoffs ist und P ein Permeabilitätskoeffizient, der stoffspezifisch ist für das Material des Packmittels und das Permeat. Somit ist die Gesamtmenge an Permeat Q linear abhängig von der Differenz des Testgaspartialdrucks außerhalb und innerhalb des Packmittels. Die Konzentration bzw. der Partialdruck des Testgases, z.B. $O_2$ innerhalb der Packung wird vernachlässigt, so dass die Partialdruckdifferenz gleich dem absoluten Druck des Testgases, d.h. hier des Sauerstoffes, außerhalb der Packung ist. In Normalatmosphäre (T = 1013 mbar) beträgt der Partialdruck an Sauerstoff P ($O_2$) = 0,21222235 bar. Somit kann man durch Erhöhung des Partialdrucks die gleiche Menge an permeierendem Testgas, hier Sauerstoff, in kürzerer Zeit erhalten. Dieser lineare Zusammenhang gilt bei konstanter Temperatur. Erhöht man beispielsweise den Partialdruck für ein ideales Testgas (z.B. 100% Sauerstoff) um den Faktor 38,6, so dass $\Delta p$ = 8,1 bar, so kann der Echtlagerzeitraum $T_{echt}$ von beispielsweise 9 Monaten auf 7 Tage reduziert werden.

Der Grad der forcierten Permeation ist dabei linear abhängig von der realen Permeabilität des Packmittels.

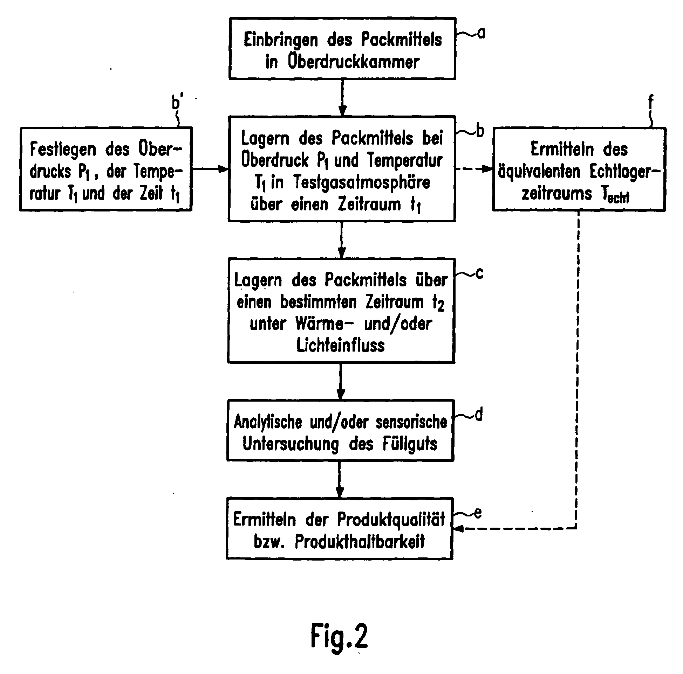

Nachfolgend wird das erfindungsgemäße Verfahren unter Bezugnahme der Figur 2 näher erläutert.

Zunächst wird das gefüllte und verschlossene Packmittel 4, hier die PET-Flaschen 4, in die Überdruckkammer 1 eingebracht und die Einbringöffnung 9 druckdicht verschlossen (Schritt a). Vor der Lagerung des Packmittels in der Überdruckkammer 1 wurden die entsprechenden Testparameter bestimmt, wobei die Höhe des Überdrucks $P_1$, die Temperatur $T_1$ und die Zeit $t_1$ zur Lagerung des Packmittels 4 in der Testgasatmosphäre festgelegt wurden (Schritt b'). In der Überdruckkammer 1 wird dann durch die Einrichtung 10, 6, 2, 7 zum Einstellen und Halten des Überdrucks $p_1$ der bestimmte Überdruck p1 eingestellt. Weiter wird eine bestimmte Temperatur $T_1$ über die Heizeinrichtung 5 eingestellt, sowie eine bestimmte relative Luftfeuchte über die Einrichtung zum Einstellen der relativen Luftfeuchtigkeit 11. Die Temperatur, der Überdruck sowie die Luftfeuchte werden über die gesamte Testdauer $t_1$ konstant gehalten. Durch die Lagerung unter Überdruck $P_1$ wird, wie zuvor erläutert, der Permeationsvorgang durch das Packmittel 4 zeitlich forciert. Dabei liegt die Temperatur $T_1$ vorzugsweise in einem Bereich von 15 bis 25 ° C, der Überdruck $p_1$ in einem Bereich von 0,1 bis 2,5 MPa, die Zeit $t_1$ in einem Bereich von 0 - 240 Stunden und die relative Luftfeuchtigkeit in einem Bereich von 0 bis 100 %.

Nach Ablauf des Zeitraums $t_1$ wird der Druck in der Überdruckkammer 1 auf Umgebungsdruck herabgesetzt und das Packmittel 4 aus der Überdruckkammer 1 entnommen.

In Schritt c) wird nun das Packmittel 4 über einen bestimmten Zeitraum $t_2$, der beispielsweise 48 bis 720 Stunden beträgt, unter Wärme- und/oder Lichteinfluss gelagert. Der Schritt c) ist notwendig, damit Interaktionen des durch die Forcierung in größerem Maße vorliegenden Testgasgehaltes mit anderen Inhaltsstoffen der Produkte beschleunigt und verstärkt werden.

Bei einer Lagerung in Dunkelheit in einem Temperaturbereich von 25 bis 60° C, bei Umgebungsdruck über einen Zeitraum von 48 bis 720 Stunden können im Falle von Sauerstoff als Testgas sauerstoffbedingte Reaktionen ablaufen, die den sauerstoffbedingten Reaktionen bei Normallagerung (Lagerung in einem mehrmonatigen äquivalenten Echtlagerzeitraum $t_{echt}$ bei Umgebungsdruck und Umgebungstemperatur) entsprechen. Somit kann eine realistische Simulation einer mehrmonatigen Lagerung erzeugt werden.

Gleichzeitig oder alternativ dazu können im Schritt c) die Packmittel 4 einer separaten Lichtbehandlung unterzogen werden, um auch Aromaveränderungen, die durch die kombinierte Einwirkung von Licht und Testgas, hier Sauerstoff, entstehen. Dabei werden die Packmittel über einen Zeitraum von 1 bis 10 Stunden einer konstanten Lichtintensität von 50 bis 750 W/m$^2$ ausgesetzt. Dies kann beispielsweise durch ein standardisierendes Testgerät, vorzugsweise Suntest XLS+ von Atlas Material Testing Technology, oder aber durch Bestrahlung der Proben mit Neonlicht in kurzer Distanz.

Im Anschluss an den Schritt c) kann nun die analytische und/oder sensorische Bewegung des Füllgutes erfolgen. Bei der analytischen und/oder sensorischen Bewertung des Füllgutes wird das Füllgut mit einer Vergleichsprobe verglichen, die in eine impermeable Verpackung gefüllt und bis zum Testzeitpunkt kühl (T = 1 bis 10° C) und dunkel gelagert wurde.

Bei der sensorischen Bewertung des Füllgutes werden die Proben z.B. in einem "Difference from Control-Test" gegen den zuvor genannten Standard, d.h. die Vergleichsprobe verkostet und die Abweichung vom Standard bewertet.

Die Produktveränderungen während des Testverfahrens können auch analytisch bewertet werden, wobei beispielsweise der Gehalt an L-Ascorbinsäure oder der Gehalt an für das Füllgut relevanten Aromastoffen beispielsweise durch HPLC- oder GC-Verfahren quantifiziert und mit dem zuvor genannten Standard verglichen wird. Ebenso möglich ist ein Aromaprofiling, wobei mittels GC-Verfahren ein Aromaprofil einer Referenzprobe, bei der das Füllgut wie zuvor beschrieben in eine impermeable Verpackung gefüllt, kühl und dunkel gelagert wurde, erstellt und mit dem Aromaprofil des Füllgutes, das gemäß dem vorliegenden Verfahren behandelt wurde, verglichen wurde.

Es kann dann aus der analytischen und/oder sensorischen Untersuchung des Füllgutes eine Aussage über die Produktqualität getroffen werden, die Rückschlüsse auf den Mindesthaltbarkeitszeitraum erlaubt.

Bei dem erfindungsgemäßen Verfahren kann entweder die Mindesthaltbarkeit bestimmt werden, indem dann ausgehend von dem Zeitraum $t_1$ ein zum Zeitraum $t_1$ äquivalenter Echtlagerzeitraum $t_{echt}$ (Schritt f) berechnet wird, in dem bei Umgebungsdruck (Normaldruck) die gleiche definierte Menge Q an Testgas in das Packmittel permeieren würde, bei der die ermittelte Produktqualität noch ausreichend ist.

Alternativ dazu können auch in Schritt b') bereits die einzelnen Paramter, wie Überdruck $p_1$, Temperatur $T_1$ und Zeit $t_1$ so berechnet werden, dass dann die Menge Q an Testgas, die in das Packmittel 4 permeieren wird, der Menge Q entspricht, die im Echtzeittest während des Echtlagerzeitraums $t_{echt}$ bei Umgebungsdruck bei entsprechender Temperatur in das Packmittel permeieren würde. In Schritt e) kann dann die Produktqualität (in Abhängigkeit der analytischen und sensorischen Untersuchungen des Füllgutes, die in Schritt d) durchgeführt wurden), ermittelt werden, so dass beispielsweise eine Aussage gemacht werden kann, ob ein bestimmtes Packmittel für ein bestehendes Produkt mit einer bestimmten Produkthaltbarkeit geeignet ist oder umgekehrt die Möglichkeit besteht, für ein bestehendes Packmittel.aus einer Anzahl von Produktvarianten die am besten geeignete auszuwählen. Weiterhin könnte beispielsweise ein orientierender Vergleich von Produktneuentwicklungen mit gängigen Marktmustem (Benchmarks) stattfinden.

Die Berechnung der entsprechenden Parameter erfolgt beispielsweise über die Gleichung (1).

Da bei karbonisiertem Füllgut im Schritt b) der $CO_2$-Gehalt während des Zeitraums $t_1$ in geringerem Maße abnimmt als im äquivalenten Echtlagerzeitraum $t_{echt}$, wird der $CO_2$-Gehalt $C_{Ziel}$ zwischen Schritt c) und d) derart eingestellt, dass er dem $CO_2$-Gehalt $C_{echt}$ entspricht, den das Füllgut nach Lagerung im Echtzeitlagerzeitraum $t_{echt}$ hätte. Der entsprechende $CO_2$-Gehalt wird in hinreichender Näherung zum Beispiel nach Gleichung (2) ermittelt.

$$C_{echt} = P\, V^{-1}\, A\, t_{echt}$$

P = Permeabilitätskoeffizient eines Kunststofffilms gegebener Dicke gegenüber dem Testgas für eine Temperatur von T = 20°C [g m$^{-2}$ bar$^{-1}$ d$^{-1}$]

A = Flaschenoberfläche [m$^2$]

V = Flaschennennvolumen [l]

$t_{echt}$ = Echtzeit [d]

Der Gehalt an $CO_2$ im Produkt ist zum Gleichgewichtsdruck im Produktspiegel proportional. Die Einstellung erfolgt daher bei Kunststoffflaschen über eine Flaschenanstichap paratur, die über ein Ventil das kontrollierte Ablassen des an einem Manometer abzulesenden Gleichgewichtsdrucks ermöglicht.

Der Ausgleich des $CO_2$-Gehaltes an den rechnerisch zu erwartenden Wert hat lediglich Einfluss auf den sensorischen Gesamteindruck und hat keinen Einfluss auf etwaige Aromaveränderungen, die analytisch detektiert werden. Eine Genauigkeit von +- 0,3 g/l ist daher hinreichend.

Zusammenfassend soll festgestellt werden, dass mit dem erfindungsgemäßen Verfahren langwierige Echtzeittests zur Bestimmung der Produktqualität im Hinblick auf Permeationsvorgänge überflüssig geworden sind. Durch die kombinierte Lagerung des Packmittels bei Überdruck mit einer Nachlagerung des Packmittels unter Wärme- und/oder Lichteinfluss kann eine realistische Simulation der Permeation in das Packmittel realisiert werden.

**Patentansprüche**

1. Verfahren zur Bewertung der Produkthaltbarkeit in einem Packmittel mit folgenden Schritten:

   a) Einbringen eines mit Füllgut gefüllten, verschlossenen Packmittels (4) in eine Überdruckkammer (1),
   b) Lagern des Packmittels (4) in der Überdruckkammer (1) über einen Zeitraum $t_1$ bei Überdruck $p_1$ und einer bestimmten Temperatur $T_1$ in einer Testgasatmosphäre, wodurch eine definierte Menge Q an Testgas in das Packmittel permeiert,
   c) Lagern des Packmittels (4) über einen bestimmten Zeitraum $t_2$ unter Wärme und/oder Lichteinfluss, und
   d) analytische und/oder sensorische Untersuchung des Füllguts.

2. Verfahren nach Anspruch 1,
   **dadurch gekennzeichnet, dass**
   das Testgas $O_2$ umfasst oder aus reinem $O_2$ besteht.

3. Verfahren nach mindestens einem der Ansprüche 1 oder 2,
   **dadurch gekennzeichnet, dass**
   die Temperatur $T_1$ in Schritt b) in einem Bereich von 5°C bis 40° C, vorzugsweise bei Zimmertemperatur in einem Bereich von 15 bis 25°C liegt.

4. Verfahren nach mindestens einem der vorhergehenden Ansprüche,
**dadurch gekennzeichnet, dass**
in Schritt b) der Überdruck $p_1$ in einem Bereich von 0,1 bis 2,5 MPa liegt.

5. Verfahren nach mindestens einem der vorhergehenden Ansprüche,
**dadurch gekennzeichnet, dass**
in Schritt b) in der Überdruckkammer (1) ein relativer Feuchtigkeitsgehalt eingestellt wird, der in einem Bereich von 0 bis 100 % liegt.

6. Verfahren nach mindestens einem der vorhergehenden Ansprüche,
**dadurch gekennzeichnet, dass**
die Temperatur $T_2$ in Schritt c) in einem Bereich von 25 bis 60° C liegt.

7. Verfahren nach mindestens einem der vorhergehenden Ansprüche,
**dadurch gekennzeichnet, dass**
zusätzlich ein zu dem Zeitraum $t_1$ äquivalenter Echtlagerzeitraum $t_{echt}$ ermittelt wird, in dem bei Umgebungsdruck (Normaldruck) $P_{norm}$ die gleiche definierte Menge Q an Testgas in das Packmittel (4) permeiert.

8. Verfahren nach Anspruch 1,
**dadurch gekennzeichnet, dass**
für Schritt b) die Zeit $t_1$ und der Überdruck $p_1$ sowie die Temperatur $T_1$ so festgelegt werden, dass eine definierte Menge Q an Testgas in das Packmittel (4) permeiert, die der Menge $Q_{echt}$ entspricht, die bei Umgebungsdruck (Normaldruck) $P_{norm}$ in Echtzeit in das Packmittel permeiert.

9. Verfahren nach mindestens einem der vorhergehenden Ansprüche,
**dadurch gekennzeichnet, dass**
das Packmittel eine Kunststoff-Flasche ist.

10. Verfahren nach mindestens einem der vorhergehenden Ansprüche,
**dadurch gekennzeichnet, dass**
in Schritt c) der Zeitraum $t_2$ in einem Bereich von 48 bis 720 Stunden liegt.

11. Verfahren nach mindestens einem der vorhergehenden Ansprüche,
**dadurch gekennzeichnet, dass**
nach Schritt c) bei karbonisiertem Füllgut der $CO_2$-Gehalt des Füllguts derart eingestellt wird, dass er dem $CO_2$-Gehalt entspricht, den das Füllgut nach Lagerung im Echtzeitlagerzeitraum $t_{echt}$ hätte.

12. Verwendung einer Vorrichtung (10) zum Beschleunigen der Permeation eines Testgases in ein Packmittel (4), das mit Füllgut gefüllt und verschlossen ist, zur Bewertung der Produkthaltbarkeit mittels eines Verfahrens nach mindestens einem der Ansprüche 1 bis 11, wobei die Vorrichtung Folgendes umfasst:

- einen Überdruckbehälter (1) zur Aufnahme von mindestens einem Packmittel (4) mit einer verschließbaren Einbringöffnung (9),
- einen Gaseingang (2) zum Zuführen des Testgases,
- einen Gasausgang (3) zum Ableiten des Testgases, und
- eine Einrichtung (10, 6, 2, 7) zum Einstellen und Halten eines Überdrucks ($P_1$).

**Claims**

1. Method for the determination of the product shelf life in a package with the following steps:

    a) placement of a closed package (4), filled with the charged product, in an overpressure chamber (1);
    b) storage of the package (4) in the overpressure chamber (1) over a time period $t_1$ at overpressure $p_1$ and at a certain temperature $T_1$ in a test gas atmosphere, wherein a defined amount Q of the test gas permeates into the package;
    c) storage of the package (4) over a certain time period $t_2$ under the influence of heat and / or light; and
    d) analytical and / or sensory examination of the charged product.

2. Method according to Claim 1,
**characterised in that**
the test gas comprises $O_2$ or consists of pure $O_2$.

3. Method according to at least one of the Claims 1 or 2,
**characterised in that**
the temperature $T_1$ in step b) lies in a range from 5°C to 40°C, preferably at room temperature in a range from 15 to 25°C.

4. Method according to at least one of the previous claims,
**characterised in that**
in step b) the overpressure $p_1$ lies in a range from 0.1 to 2.5 MPa.

5. Method according to at least one of the previous claims,
**characterised in that**
in step b) in the overpressure chamber (1) a relative moisture content is set which lies in a range from 0 to 100%.

**6.** Method according to at least one of the previous claims,
**characterised in that**
the temperature $T_2$ in step c) lies in a range from 25 to 60°C.

**7.** Method according to at least one of the previous claims,
**characterised in that**
in addition a real storage time period $t_{real}$ equivalent to the time period $t_1$ is determined in which the same defined amount Q of the test gas permeates into the package (4) at ambient pressure (standard pressure) $P_{standard}$.

**8.** Method according to Claim 1,
**characterised in that**
for step b) the time $t_1$ and the overpressure $p_1$ as well as the temperature $T_1$ are established such that a defined amount Q of test gas permeates into the package (4), which corresponds to the amount $Q_{real}$, which permeates into the package in real time at ambient pressure (standard pressure) $P_{standard}$.

**9.** Method according to at least one of the previous claims,
**characterised in that**
the package is a plastic bottle.

**10.** Method according to at least one of the previous claims,
**characterised in that**
in step c) the time period $t_2$ lies in a range from 48 to 720 hours.

**11.** Method according to at least one of the previous claims,
**characterised in that**
after step c) with carbonised charged product, the $CO_2$ content of the charged product is set such that it corresponds to the $CO_2$ content which the charged product would have after storage over the real-time storage period $t_{real}$.

**12.** Use of a device (10) for the acceleration of the permeation of a test gas into a package (4), which is filled with the charged product and closed, for the determination of the product shelf life by means of a method according to at least one of the Claims 1 to 11, wherein the device comprises the following:

- an overpressure container (1) for the accommodation of at least one package (4) with a closable inlet opening (9),
- a gas inlet (2) for feeding in the test gas,
- a gas outlet (3) for discharging the test gas, and
- a facility (10, 6, 2, 7) for setting and holding an overpressure ($P_1$).

**Revendications**

**1.** Procédé pour déterminer la durée de conservation d'un produit emballé, comportant les étapes consistant à :

a) apporter un emballage de produit (4) rempli d'un produit, fermé, dans une chambre de surpression (1) ;
b) déposer le produit emballé (4) dans la chambre de surpression (1) pendant une durée $t_1$ sous une surpression $p_1$ et une température déterminée $T_1$ dans une atmosphère de gaz d'essai, permettant à une quantité définie Q de gaz d'essai de pénétrer dans le produit emballé ;
c) déposer le produit emballé (4) pendant une durée $t_2$ sous une entrée de chaleur et/ou de lumière, et
d) faire une recherche analytique et/ou sensorielle sur le produit emballé.

**2.** Procédé selon la revendication 1, **caractérisé en ce que** le gaz d'essai comprend de l'$O_2$ ou est constitué d'$O_2$ pur.

**3.** Procédé selon l'une au moins des revendications 1 et 2, **caractérisé en ce que** la température $T_1$ de l'étape b) est comprise dans une plage de 5°C à 40°C, de préférence avec une température de la chambre dans une plage de 15°C à 25°C.

**4.** Procédé selon l'une au moins des revendications précédentes, **caractérisé en ce que** lors de l'étape b), la surpression $p_1$ est comprise dans une plage de 0,1 à 2,5 MPa.

**5.** Procédé selon l'une au moins des revendications précédentes, **caractérisé en ce que** lors de l'étape b), une teneur en humidité relative dans la chambre de surpression (1) est instaurée, comprise dans une plage de 0 à 100 %.

**6.** Procédé selon l'une au moins des revendications précédentes, **caractérisé en ce que** la température $T_2$ lors de l'étape c) est comprise dans une plage de 25 à 60°C.

**7.** Procédé selon l'une au moins des revendications précédentes, **caractérisé en ce qu'**une durée de dépôt réelle $t_{réel}$ équivalente à la durée $t_1$ est en outre déterminée, pendant laquelle, sous une pression ambiante (pression normale) $P_{norm}$, la quantité définie égale Q de gaz d'essai pénètre dans le produit emballé (4).

**8.** Procédé selon la revendication 1, **caractérisé en ce que** pour l'étape b), la durée $t_1$ et la surpression $p_1$ ainsi que la température $T_1$ sont réglées de telle manière qu'une quantité définie Q de gaz d'essai pénètre dans le produit emballé (4), correspondant à la quantité $Q_{réel}$ qui pénètre sous une pression ambiante (pression normale) $P_{norm}$ en temps réel dans le produit emballé.

9. Procédé selon l'une au moins des revendications précédentes, **caractérisé en ce que** le produit emballé est une bouteille en plastique.

10. Procédé selon l'une au moins des revendications précédentes, **caractérisé en ce que** dans l'étape c), la durée $t_2$ est comprise dans une plage de 48 à 720 heures.

11. Procédé selon l'une au moins des revendications précédentes, **caractérisé en ce que** après l'étape c), la teneur en $CO_2$ du produit dans le produit carbonisé est ajustée de telle manière qu'elle correspond à la teneur en $CO_2$ que le produit aurait après le dépôt pendant la durée de dépôt réelle $t_{réel}$.

12. Utilisation d'un dispositif (10) accélération de la pénétration d'un gaz d'essai dans un produit emballé (4), rempli du produit et fermé, pour déterminer la durée de conservation du produit au moyen d'un procédé selon l'une au moins des revendications 1 à 11, dans lequel le dispositif comprend les éléments suivants :

   - un réservoir de surpression (1) pour la réception d'au moins un produit emballé (4) comportant une ouverture d'entrée refermable (9),
   - une entrée de gaz (2) pour l'alimentation en gaz d'essai,
   - une sortie de gaz (3) pour l'évacuation du gaz d'essai, et
   - un dispositif (10, 6, 2, 7) pour l'instauration et le maintien d'une surpression ($P_1$).

**Fig.1**

Einbringen des Packmittels
in Überdruckkammer — a

Festlegen des Oberdrucks $P_1$, der Temperatur $T_1$ und der Zeit $t_1$ — b'

Lagern des Packmittels bei
Überdruck $P_1$ und Temperatur
$T_1$ in Testgasatmosphäre
über einen Zeitraum $t_1$ — b

Ermitteln des
äquivalenten Echtlagerzeitraums $T_{echt}$ — f

Lagern des Packmittels über
einen bestimmten Zeitraum $t_2$
unter Wärme- und/oder
Lichteinfluss — c

Analytische und/oder sensorische
Untersuchung des Füllguts — d

Ermitteln der Produktqualität
bzw. Produkthaltbarkeit — e

## Fig.2

**EP 1 626 275 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2004049372 A1